(19) Europäisches Patentamt
European Patent Office
Office européen des brevets

(11) **EP 4 718 468 A1**

(12) **EUROPEAN PATENT APPLICATION**

(43) Date of publication:
**01.04.2026 Bulletin 2026/14**

(21) Application number: **24465579.1**

(22) Date of filing: **27.09.2024**

(51) International Patent Classification (IPC):
***G16H 50/20*** (2018.01)   ***G16H 30/20*** (2018.01)

(52) Cooperative Patent Classification (CPC):
**G16H 30/20; G16H 50/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **SERBAN, Alexandru Constantin**
**900520 Constanta (RO)**
• **GULSUN, Mehmet Akif**
**Princeton, NJ 08540 (US)**
• **SHARMA, Puneet**
**Princeton Junction, NJ 08550 (US)**
• **COMANICIU, Dorin**
**Princeton, NJ 08540 (US)**

(74) Representative: **HKW Intellectual Property PartG mbB**
**Theresienhöhe 12**
**80339 München (DE)**

(54) **MULTI-MODAL MULTI-TASK FOUNDATIONAL MODELS FOR MEDICAL IMAGE MANIPULATION AND INFORMATION RETRIEVAL**

(57)     Systems and methods for automatically performing one or more actions on one or more medical applications are provided. Text-based instructions are received. The text-based instructions are encoded into text features using a machine learning based text encoder network. One or more instructions for performing by one or more medical applications are determined using a policy module based on the text features. The one or more instructions are performed by the one or more medical applications to generate a response to the text-based instructions. The response to the text-based instructions is output.

**FIG. 1**     100

Receive text-based instructions and one or more medical images
102

Encode the text-based instructions into text features using a machine learning based text encoder network
104

Encode the one or more medical images into image features using a machine learning based image encoder network
106

Determine one or more instructions for performing by one or more medical applications using a policy module based on the text features and the image features
108

Perform the one or more instructions by the one or more medical applications to generate a response to the text-based instructions
110

Output the response to the text-based instructions
112

## Description

TECHNICAL FIELD

The present invention relates generally to AI/ML (artificial intelligence/machine learning) based foundational models, and, in particular, to multi-modal multi-task foundational models for medical image manipulation and information retrieval.

BACKGROUND

During interventional procedures, clinicians interact with imaging devices, patient databases, and other medical devices using dedicated interface devices, such as, e.g., keyboards, mice, and touchscreens. However, interaction during interventional procedures using such dedicated interface devices may be cumbersome, relatively timeconsuming, and distracts the clinicians from focusing on the interventional procedures. Recently, voice-based interface devices have been proposed for interacting with medical devices using natural language processing. However, conventional voice-based interface devices do not enable precise control of medical devices. For example, a clinician can rotate a medical image using a mouse clockwise by 10 degrees but the command "rotate the image slightly clockwise" may not provide the desired rotation.

BRIEF SUMMARY OF THE INVENTION

In accordance with one or more embodiments, systems and methods for automatically performing one or more instructions by one or more medical applications are provided. Text-based instructions are received. The text-based instructions are encoded into text features using a machine learning based text encoder network. One or more instructions for performing by one or more medical applications are determined using a policy module based on the text features. The one or more instructions are performed by the one or more medical applications to generate a response to the text-based instructions. The response to the text-based instructions is output.

In one embodiment, one or more medical images are received. The one or more medical images are encoded into image features using a machine learning based image encoder network. The one or more instructions are determined further based on the image features. The one or more instructions are performed by the one or more medical applications to modify the one or more medical images. The one or more modified medical images are output. The machine learning based text encoder network and the machine learning based image encoder network are trained to generate similar features for associated text-based instructions and medical images.

In one embodiment, the policy module is adapted based on user feedback to the response to the text-based instructions.

In one embodiment, the one or more instructions comprise at least one of: one or more medical image analysis tasks performed on the one or more medical images, functions to derive findings from the one or more medical images, functions to apply transformations on the one or more medical images, functions to derive information from the one or more medical images, or outputting text to a machine learning based model.

In one embodiment, the one or more instructions comprise one or more APIs (application programming interfaces).

In one embodiment, spoken instructions are received from a user. The spoken instructions are converted to the text-based instructions.

In one embodiment, the machine learning based text encoder network comprises a language model (e.g., a large language model).

These and other advantages of the invention will be apparent to those of ordinary skill in the art by reference to the following detailed description and the accompanying drawings.

BRIEF DESCRIPTION OF THE DRAWINGS

Figure 1 shows a method for performing one or more instructions by one or more medical applications based on text-based instructions, in accordance with one or more embodiments;

Figure 2 shows a workflow for performing one or more instructions by one or more medical applications based on text-based instructions, in accordance with one or more embodiments;

Figure 3 shows a framework for jointly training a machine learning based image encoder network and a machine learning based text encoder network, in accordance with one or more embodiments;

Figure 4 shows an architecture of a policy module, in accordance with one or more embodiments;
Figure 5 shows a workflow 500 for adapting a policy module, in accordance with one or more embodiments;
Figure 6 shows an exemplary artificial neural network that may be used to implement one or more embodiments;
Figure 7 shows a convolutional neural network that may be used to implement one or more embodiments;
Figure 8 shows a schematic structure of a recurrent machine learning model that may be used to implement one or more embodiments; and
Figure 9 shows a high-level block diagram of a computer that may be used to implement one or more embodiments.

DETAILED DESCRIPTION

The present invention generally relates to multi-modal multi-task foundational models for medical image manipulation and information retrieval from images. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system. Further, reference herein to pixels of an image may refer equally to voxels of an image and vice versa.

Embodiments disclosed herein provide for a multi-modal image-text foundational model to understand and execute user instructions for medical image manipulation, information retrieval, and other medical tasks. The foundational model comprises a machine learning based text encoder network and a machine learning based image encoder network for understanding and decomposing natural language text-based instructions and one or more medical images respectively. A policy module maps the features extracted by the foundational model to one or more instructions for generating a response to the text-based instructions with image and/or text data for real-time, joint manipulation of medical images and information retrieval. Advantageously, embodiments described herein facilitate user interface interactions with medical applications by learning to perform actions that satisfy information needs for both medical images and text.

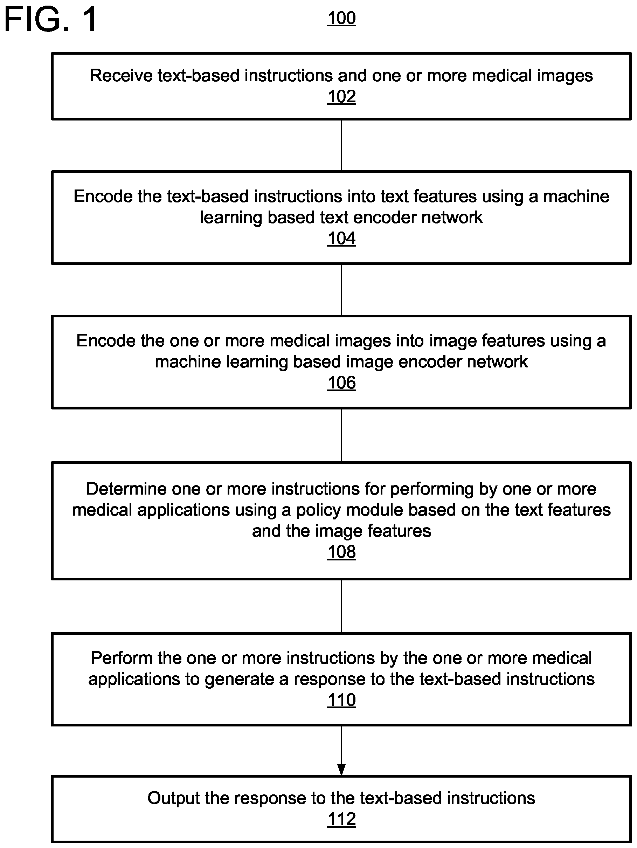

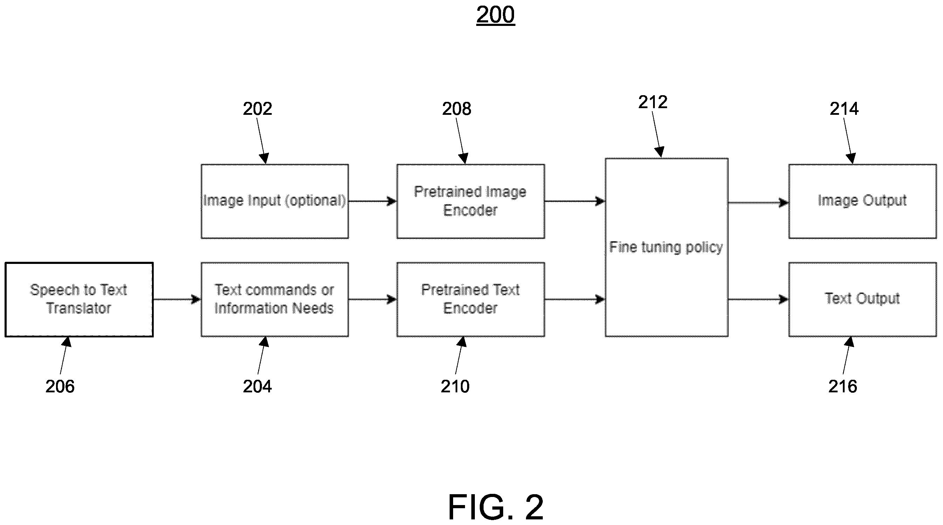

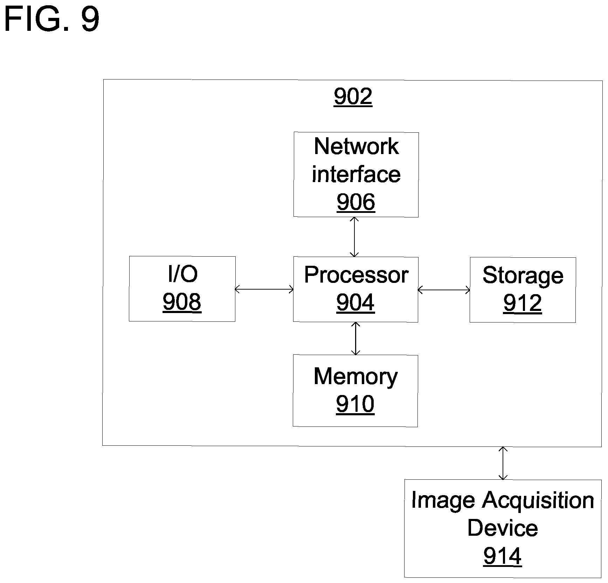

Figure 1 shows a method 100 for performing one or more instructions by one or more medical applications based on text-based instructions, in accordance with one or more embodiments. The steps and sub-steps of method 100 may be performed by one or more suitable computing devices, such as, e.g., computer 902 of Figure 9. Figure 2 shows a workflow 200 for performing one or more instructions by one or more medical applications based on text-based instructions, in accordance with one or more embodiments. Figure 1 and Figure 2 will be described together.

At step 102 of Figure 1, text-based instructions and (optionally) one or more medical images are received. In one example, as shown in workflow 200 of Figure 2, the text-based instructions are text commands or information needs 204 and the one or more medical images is image input 202.

The text-based instructions may comprise natural language text-based commands or queries for interacting with one or more medical applications, such as, e.g., medical imaging applications for viewing and analyzing medical images, patient databases for retrieving and analyzing medical data, machine learning based models for performing medical analysis tasks, etc. For example, the text-based instructions may be "rotate the medical images clockwise a little bit" or "how big is the lung lesion". In one embodiment, spoken instructions are first received from a clinician or other user (e.g., via a microphone) and the spoken instructions are converted to the text-based instructions using, e.g., any well-known speech-to-text translator. For example, as shown in workflow 200 of Figure 2, speech may be received by speech to text translator 206, which converts the speech to text commands or information needs 204.

The one or more medical images may depict an anatomical object, such as, e.g., organs, bones, vessels, tumors or other abnormalities, or any other anatomical object of interest of a patient. The one or more medical images are associated with the text-based instructions. For example, the text-based instructions may be instructions for modifying, extracting information from, or otherwise analyzing the one or more medical images. The one or more medical images may be of any suitable modality, such as, e.g., MRI (magnetic resonance imaging), PET (positron emission tomography), SPECT (single photon emission computed tomography), CT (computed tomography), US (ultrasound), X-ray, or any other medical imaging modality or combinations of medical imaging modalities. The one or more medical images may be 2D (two dimensional) images and/or 3D (three dimensional) volumes and may comprise a single image or a plurality of images.

The text-based instructions and/or one or more medical images may be received, for example, by directly receiving the text-based instructions from a user via an input/output (I/O) device (e.g., I/O 908 of Figure 9), by directly receiving the one or more medical images from an medical image acquisition device (e.g., image acquisition device 914 of Figure 9) as the images are acquired, by loading the text-based instructions and/or one or more medical images from a storage or memory of a computer system (e.g., storage 912 or memory 910 of computer 902 of Figure 9), or by receiving the text-based instructions and/or one or more medical images from a remote computer system (e.g., computer 902 of Figure 9). Such a computer system or remote computer system may comprise one or more patient databases, such as, e.g. , an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 104 of Figure 1, the text-based instructions are encoded into text features using a machine learning based text encoder network and, (optionally) at step 106 of Figure 1, the one or more medical images are encoded into image features using a machine learning based image encoder network. In one example, as shown in workflow 200 of Figure 2, text commands or information needs 204 are encoded into text features by the pretrained text encoder 210 and, optionally, the image input is encoded into image features by pretrained image encoder 208. The text features and image features are compact, fixed-size representations (e.g., vectors) of the text-based instructions and the one or more medical images respectively.

The machine learning based image encoder network receives as input the one or more medical images and generates as output the image features. The machine learning based image encoder network may be implemented according to any suitable machine learning based architecture, such as, e.g., an autoencoder, a vision transformer, a CNN (convolutional neural network), etc. The machine learning based text encoder network receives as input the text-based instructions and generates as output the text features. The machine learning based text encoder network may be implemented according to any suitable machine learning based architecture. In one embodiment, the machine learning based text encoder network is a language model, such as, e.g., an LLM (large language model). However, the language model may be any other suitable language model. For example, the language model may be a small language model, which uses a relatively smaller neural network, has fewer parameters, and is trained on less training data as compared with an LLM.

The LLM may be any suitable pretrained deep learning based LLM. The LLM acts as an embedding language system for generating relevant vectors from a query. For example, the LLM may be based on the transformer architecture, which uses an attention mechanism to capture long-range dependencies in text. Exemplary transformer-based architectures include Med-PaLM (Medical Pathway Language Model), BLOOM (BigScience Large Open-science Open-access Multilingual Language Model), and BERT (Bidirectional Encoder Representations from Transformers).

The machine learning based text encoder network and the machine learning based image encoder network together form a multi-modal image-text foundational model. In one embodiment, the multi-modal image-text foundational model may be any well-known, off-the-shelf multi-modal image-text foundational model. For example, the multi-modal image-text foundational model may be BiomedCLIP.

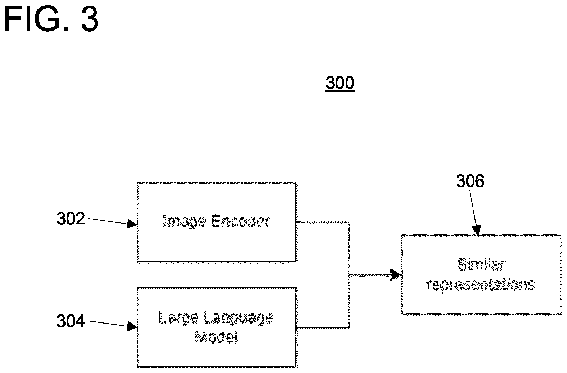

The machine learning based text encoder network and the machine learning based image encoder network are jointly trained during a prior offline or training stage to generate same or similar features for the associated text-based instructions and one or more medical images. In one embodiment, the machine learning based text encoder network and the machine learning based image encoder network are trained according to Figure 3, described in detail below. Once trained, the machine learning based text encoder network and/or the machine learning based image encoder network are applied during an online or inference stage, e.g., to perform steps 104 and 106 of Figure 1.

Figure 3 shows a framework 300 for jointly training a machine learning based image encoder network and a machine learning based text encoder network, in accordance with one or more embodiments. In framework 300, the machine learning based text encoder is large language model 304. Large language model 304 may be the machine learning based text encoder network utilized at step 106 of Figure 1 or pretrained text encoder 210 of Figure 2. Image encoder 302 may be the machine learning based image encoder network utilized at step 108 of Figure 1 or pretrained image encoder 208 of Figure 2.

Image encoder 302 and large language model 304 are jointly trained to generate same or similar representations 306 (or features) for associated text-based instructions and medical images. In one embodiment, image encoder 302 and large language model 304 are trained according to CLIP (contrastive language image pretraining) using associated training text-based instructions and training medical images. Image encoder 302 receives the training medical images as input and generates image features as output. Large language model 304 receives the training text-based instructions as input and generates text features as output. In one embodiment, image encoder 302 and large language model 304 are trained using contrastive learning using pairs of similar and dissimilar training medical images/training text-based instructions. Given associated training text-based instructions and training medical images, image encoder 302 and large language model 304 are trained to maximize the similarity (e.g., measured by a dot product) between the image features and text features, while minimizing the similarity between the image features and text features extracted from randomly sampled text-based instructions.

Referring back to Figure 1, at step 108, one or more instructions for performing by one or more medical applications are determined using a policy module based on the text features and (optionally) the image features. In one example, the policy module is fine tuning policy module 212 of Figure 2. The policy module maps the text and/or image features into one or more instructions according to a policy to generate a response to the text-based instructions. For example, an instruction may be defined for rotating (or otherwise modifying) an image at a desired angle and the outcome may be the rotated image.

The one or more instructions may comprise actions, guidance, or any other suitable instructions. In one embodiment, the one or more instructions comprise at least one of: 1) one or more medical image analysis tasks performed on the one or more medical images (e.g., medical image detection, classification, and segmentation using machine learning based models), 2) functions to derive measurements or other findings from the one or more medical images (e.g., stenosis measurement from coronary segmentations, CAD-RADS (coronary artery disease - reporting and data systems) findings from stenosis measurements in detected segmentations), 3) functions to apply transformations on the one or more medical images (e.g., rotate an image by a particular angle), 4) functions to derive information from the one or more medical images (e.g., whether the specific organ is blocked in a given camera angle), or 5) outputting text to another machine learning based network or medical application (e.g., an agent that can fetch patient's clinical reports and parse actionable information from it). In one embodiment, the one or more actions comprise one or more APIs (application programming interfaces) for communicating with the one or more medical applications.

The policy module is trained during a prior offline or training stage together with the machine learning based text encoder network and the machine learning based image encoder network. During training, the one or more instructions are defined in text, together with thein input and output parameters and a short text description of their scope. The definitions of the one or more instructions may be input to the policy module, or may be combined with training text-based instructions or training text-based instructions/image pairs and input to the policy module. The policy module is trained using training text-based instructions or training text-based instructions/image pair with their expected outcomes. In one embodiment, the expected outcomes may be simulated either following predefined workflows or by caching results from user interactions. Alternative text-based instructions may also be generated using a language model.

The policy module is trained to optimize the policy using policy optimization algorithms by adjusting policy parameters to maximize expected rewards. The goal of the policy module is to find a relevant set of instructions to satisfy the query. The policy module may be implemented as a neural network with parameters updated based on feedback from the environment using reinforcement learning. The policy optimization algorithms update the policy using optimization techniques, such as, e.g., gradient descent. The policy module is jointly trained with the machine learning based text encoder network and the machine learning based image encoder network.

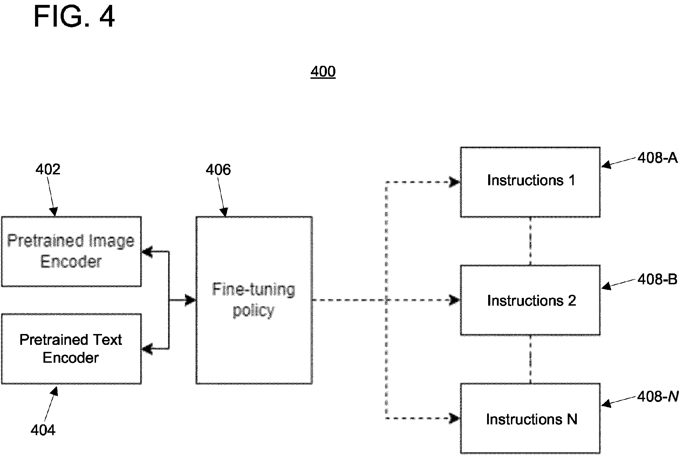

Figure 4 shows an architecture 400 of a policy module, in accordance with one or more embodiments. Policy module 406 receives image features from pretrained image encoder 402 and text features from pretrained image encoder 402 and generates instructions 408-A, 408-B, ..., 408-N.

Referring back to Figure 1, at step 110, the one or more instructions are performed by the one or more medical applications to generate a response to the text-based instructions. In one example, as shown in workflow 200 of Figure 2, the response to the text-based instructions may comprise image output 214 and/or text output 216. For example, where the one or more instructions comprise modifying the one or more medical images, the response to the text-based instructions may comprise the one or more modified medical images.

At step 112 of Figure 1, the response to the text-based instructions is output. For example, the response to the text-based instructions can be output by displaying the response on a display device of a computer system (e.g., I/O 908 of computer 902 of Figure 9), storing the response on a memory or storage of a computer system (e.g., memory 910 or storage 912 of computer 902 of Figure 9), or by transmitting the response to a remote computer system (e.g., computer 902 of Figure 9). In one embodiment, the response to the text-based instructions may be overlaid on the one or more medical images and presented via a display device.

In one embodiment, the policy module may be adapted or personalized in real-time based on user feedback to the response to the text-based instructions. For example, where the text-based instruction is "rotate the one or more medical images", the one or more rotated medical images may be presented to a user (at step 112 of Figure 1). The user feedback may comprise, e.g., additional text-based instructions (e.g., spoken instructions converted to the additional text-based instructions) to "rotate the one or more medical images a little more" or may comprise user input via one or more interface devices (e.g., mouse or keyboard) further rotating the one or more rotated medical images to a desired angle. In another example, the user feedback may comprise a request for additional information. The policy of the policy module is adapted based on the user feedback to generate an updated response to the text-based instructions. The policy module is adapted during an offline or training stage. During the adaptation of the policy module, the machine learning based text encoder network and the machine learning based text encoder network are not updated.

In one embodiment, adaption of the policy module may be repeated any number of iterations in response to a sequence of user feedback. The sequence of user feedback may be used by the policy module to map the text and/or image features to the one or more actions.

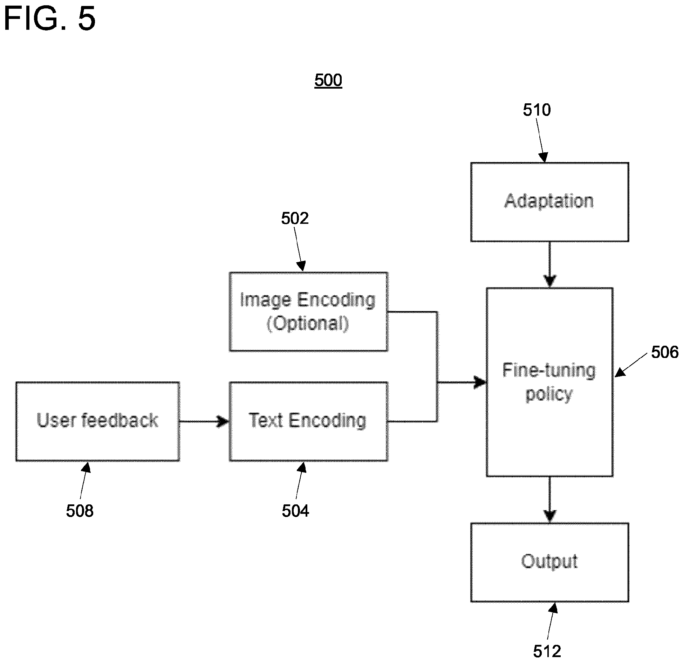

Figure 5 shows a workflow 500 for adapting a policy module, in accordance with one or more embodiments. In workflow 500, image encoding 502 may be the machine learning based image encoder network utilized at step 106 of Figure 1, pretrained image encoder 208 of Figure 2, image encoder 302 of Figure 3, or pretrained image encoder 402 of Figure 4; text encoding 504 may be machine learning based text encoder network utilized at step 104 of Figure 1, pretrained text encoder 210 of Figure 2, large language model 304 of Figure 3, or pretrained text encoder 404 of Figure 4; and fine-tuning policy module 506 may be the policy module utilized at step 108 of Figure 1, fine tuning policy module 212 of Figure 2, or fine-tuning policy module 406 of Figure 4. In response to outputting the response to the text-based instructions, user feedback 508 is received. Fine-tuning policy module 506 is updated with adaptation 510 based on user feedback 508 to generate an updated response to the text-based instructions as output 512. For each interaction, the policy keeps a set of parameters which are used to adapt/personalize the policy. Upon each interaction and depending on the user feedback, the policy only changes this set of parameters to adapt/personalize. This provides both speed and efficiency, allowing the adaptation module to run in real time.

In one exemplary scenario, embodiments described herein may be applied for an interventional cardiologist interacting with a pre-processed CTA (CT angiography) scan where the detailed coronary artery lumen, plaque, and computed flow/pressure information has already been extracted and verified in a pre-operative setting (e.g., by a radiologist).

In this exemplary scenario, a first text-based instruction may be: "What is the size of stent I need for the mid LAD lesion?" where LAD refers to left anterior descending artery. Embodiments described herein determine one or more actions to extract or retrieve information related to stenosis findings, stenosis quantification, and coronary devices (e.g., available stent sizes) to generate a response to the text-based instruction.

A second text-based instruction may be: "Would proximal one still need stenting after this?" Embodiments described herein update the context to reflect that the first lesion has already been treated and determine one or more actions to generate post-PCI (percutaneous coronary intervention) lumen and flow scenarios. Based on the updated context, one or more actions are determined to compute the CT-FFR (CT fractional flow reserve) for the proximal lesion in this updated context.

A third text-based instruction may be: "What is a good working angle for the mid RCA lesion?" where RCA refers to right coronary artery. Embodiment described herein are able to contextualize the instruction and retrieve information about the recommended C-arm angulations for a mid-RCA segment (e.g., from prior information in textbooks based on population data).

User feedback may then be received as follows: "I meant for this patient." Embodiments described herein will now determine an action that can perform 3D geometry operations to compute the 2D projection of 3D CTA centerline and determine the least foreshortened view.

Further user feedback may then be received as follows: "This one has some overlapping vessels. Get me a slightly different one." Embodiments described herein would repeatedly call the action that can perform 3D geometry operations to compute the 2D projection of 3D CTA centerline and determine the least foreshortened view, to thereby generated other views.

Further user feedback may then be received as follows: "What was the wall motion like for the mid LAD stenosis." Embodiments described herein call an action to retrieve wall motion analysis results from a prior echo study and then filter and present the results for the left ventricle wall segment that is associated with the mid LAD coronary segment.

Embodiments described herein may be applied to perform the following exemplary applications: 1) Perform a complex task by decomposing text-based instructions into a set of actions to sub-tasks; 2) Search data and parsing actional information from it by decomposing the text-based instructions into a set of actions including: a) APIs provided by the electronic health record system, b) actions for image and document classification, and c) actions for parsing information from text using language models; and 3) translating user queries into queries for data and information retrieval based on the application context or the contextual information about the user and patient.

Embodiments described herein may be implemented according to the following exemplary implementation.

Objective: zoom in on the aortic root in a CT scan.

Requirements: 1) Actions for aorta segmentation, which returns a segmentation map and the position of the aortic root. The actions must also have a textual description including the input and output parameters, and a description of its scope. 2) Actions for zooming in on one region of interest of an image, together with its description. 3) Query or visual description of the information needed. 4) A CT scan. 5) A multi-modal biomedical language model trained together with the policy module.

Inference: 1) The action descriptions are concatenated with the text-based instructions and sent to the model, which outputs a chain of actions and their parameters. 2) The actions are executed sequentially.

Adaptation: For user feedback, the previous action descriptions and text-based instructions are concatenated with the user feedback and the model outputs a new set of actions.

Training: For training, a larger set of text-based instructions and actions will be used. The model will receive the action descriptions and the text-based instructions, and will output a set of actions. For each correct action, the model receives a reward, which is cumulative in case of multiple actions. In case the model adds extra actions, it will get penalized with negative rewards. A score is computed given the rewards and the model gets optimized. In case of errors the model's parameters are adjusted. For computing the errors, the sequence of actions or the parameters of the actions may be check or data from the action calls may be simulated and data-related metrics may be computed.

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model, such as, e.g., The machine learning based text encoder network utilized at step 104, the machine learning based image encoder network utilized at step 106, and the policy module utilized at step 108 of Figure 1, pretrained image encoder 208, pretrained text encoder 210, and fine tuning policy 212 of Figure 2, image encoder 302 and large language module 204 of Figure 3, pretrained image encoder 402 and pretrained text encoder 404 of Figure 4, image encoding 501, text encoding 504, and fine-tuning policy 506 of Figure 5, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

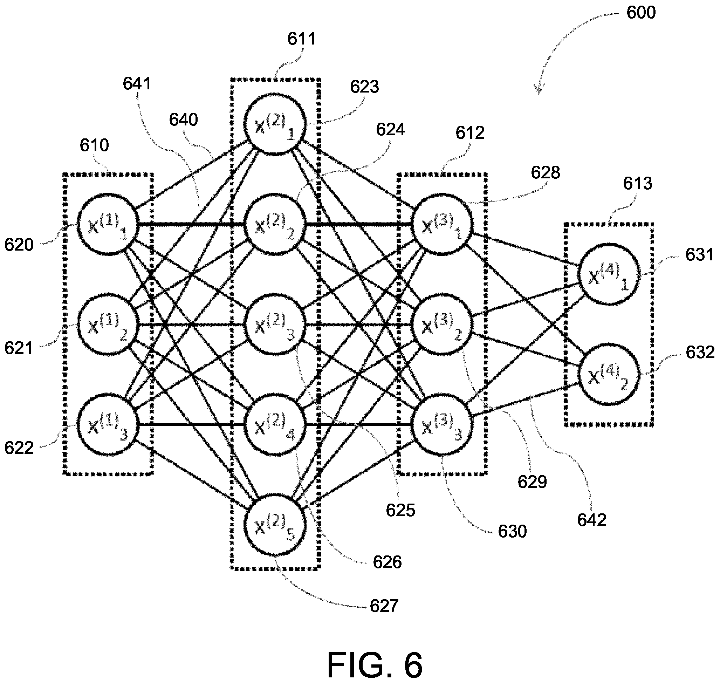

Figure 6 shows an embodiment of an artificial neural network 600 that may be used to implement one or more machine learning models described herein. Alternative terms for "artificial neural network" are "neural network", "artificial neural net" or "neural net".

The artificial neural network 600 comprises nodes 620, ..., 632 and edges 640, ..., 642, wherein each edge 640, ..., 642 is a directed connection from a first node 620, ..., 632 to a second node 620, ..., 632. In general, the first node 620, ..., 632 and the second node 620, ..., 632 are different nodes 620, ..., 632, it is also possible that the first node 620, ..., 632 and the second node 620, ..., 632 are identical. For example, in Figure 6 the edge 640 is a directed connection from the node 620 to the node 623, and the edge 642 is a directed connection from the node 630 to the node 632. An edge 640, ..., 642 from a first node 620, ..., 632 to a second node 620, ..., 632 is also denoted as "ingoing edge" for the second node 620, ..., 632 and as "outgoing edge" for the first node 620, ..., 632.

In this embodiment, the nodes 620, ..., 632 of the artificial neural network 600 can be arranged in layers 610, ..., 613, wherein the layers can comprise an intrinsic order introduced by the edges 640, ..., 642 between the nodes 620, ..., 632. In particular, edges 640, ..., 642 can exist only between neighboring layers of nodes. In the displayed embodiment, there is an input layer 610 comprising only nodes 620, ..., 622 without an incoming edge, an output layer 613 comprising only nodes 631, 632 without outgoing edges, and hidden layers 611, 612 in-between the input layer 610 and the output layer 613. In general, the number of hidden layers 611, 612 can be chosen arbitrarily. The number of nodes 620, ..., 622 within the input layer 610 usually relates to the number of input values of the neural network, and the number of nodes 631, 632 within the output layer 613 usually relates to the number of output values of the neural network.

In particular, a (real) number can be assigned as a value to every node 620, ..., 632 of the neural network 600. Here, $x^{(n)}_i$ denotes the value of the i-th node 620, ..., 632 of the n-th layer 610, ..., 613. The values of the nodes 620, ..., 622 of the input layer 610 are equivalent to the input values of the neural network 600, the values of the nodes 631, 632 of the output layer 613 are equivalent to the output value of the neural network 600. Furthermore, each edge 640, ..., 642 can comprise a weight being a real number, in particular, the weight is a real number within the interval [-1, 1] or within the interval [0, 1]. Here, $w^{(m,n)}_{i,j}$ denotes the weight of the edge between the i-th node 620, ..., 632 of the m-th layer 610, ..., 613 and the j-th node 620, ..., 632 of the n-th layer 610, ..., 613. Furthermore, the abbreviation $w^{(n)}_{i,j}$ is defined for the weight $w^{(n,n+1)}_{i,j}$.

In particular, to calculate the output values of the neural network 600, the input values are propagated through the neural network. In particular, the values of the nodes 620, ..., 632 of the (n+1)-th layer 610, ..., 613 can be calculated based on the values of the nodes 620, ..., 632 of the n-th layer 610, ..., 613 by

$$x^{(n+1)_j} = f\left(\sum_i x^{(n)_i} \cdot w^{(n)_{i,j}}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 610 are given by the input of the neural network 600, wherein values of the first hid-den layer 611 can be calculated based on the values of the input layer 610 of the neural network, wherein values of the second hidden layer 612 can be calculated based in the values of the first hidden layer 611, etc.

In order to set the values $w^{(m,n)}_{i,j}$ for the edges, the neural network 600 has to be trained using training data. In particular, training data comprises training input data and training output data (denoted as $t_i$). For a training step, the neural network 600 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 600 (backpropagation algorithm). In particular, the weights are changed according to

$$w'^{(n)_{i,j}} = w^{(n)_{i,j}} - \gamma \cdot \delta^{(n)_j} \cdot x^{(n)_i}$$

wherein $\gamma$ is a learning rate, and the numbers $\delta^{(n)}_j$ can be recursively calculated as

$$\delta^{(n)_j} = \left(\sum_k \delta^{(n+1)_k} \cdot w^{(n+1)_{j,k}}\right) \cdot f'\left(\sum_i x^{(n)_i} \cdot w^{(n)_{i,j}}\right)$$

based on $\delta^{(n+1)}_j$, if the (n+1)-th layer is not the output layer, and

$$\delta^{(n)_j} = \left(x^{(n+1)_j} - t^{(n+1)_j}\right) \cdot f'\left(x^{(n)_i} \cdot w^{(n)_{i,j}}\right)$$

if the (n+1)-th layer is the output layer 613, wherein f' is the first derivative of the activation function, and $t^{(n+1)}_j$ is the comparison training value for the j-th node of the output layer 613.

A convolutional neural network is a neural network that uses a convolution operation instead general matrix multiplication in at least one of its layers (so-called "convolutional layer"). In particular, a convolutional layer performs a dot product of one or more convolution kernels with the convolutional layer's input data / image, wherein the entries of the one or more convolution kernel are the parameters or weights that are adapted by training. In particular, one can use the Frobenius inner product and the ReLU activation function. A convolutional neural network can comprise additional layers, e.g., pooling layers, fully connected layers, and normalization layers.

By using convolutional neural networks input images can be processed in a very efficient way, because a convolution operation based on different kernels can extract various image features, so that by adapting the weights of the convolution kernel the relevant image features can be found during training. Furthermore, based on the weightsharing in the convolutional kernels less parameters need to be trained, which prevents overfitting in the training phase and allows to have faster training or more layers in the network, improving the performance of the network.

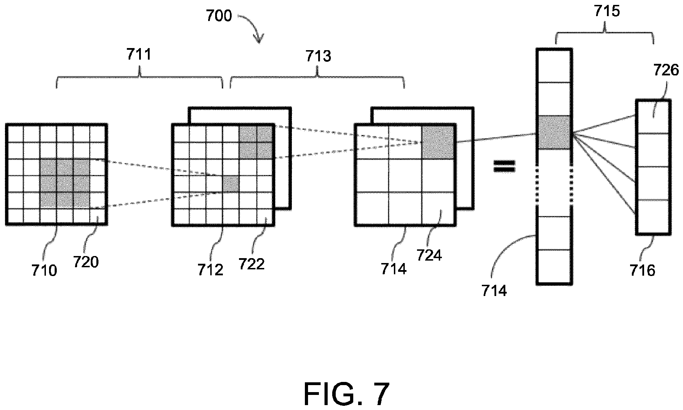

Figure 7 shows an embodiment of a convolutional neural network 700 that may be used to implement one or more machine learning models described herein. In the displayed embodiment, the convolutional neural network comprises 700 an input node layer 710, a convolutional layer 711, a pooling layer 713, a fully connected layer 714 and an output node layer 716, as well as hidden node layers 712, 714. Alternatively, the convolutional neural network 700 can comprise several convolutional layers 711, several pooling layers 713 and several fully connected layers 715, as well as other types of layers. The order of the layers can be chosen arbitrarily, usually fully connected layers 715 are used as the last layers before the output layer 716.

In particular, within a convolutional neural network 700 nodes 720, 722, 724 of a node layer 710, 712, 714 can be considered to be arranged as a d-dimensional matrix or as a d-dimensional image. In particular, in the two-dimensional case the value of the node 720, 722, 724 indexed with i and j in the n-th node layer 710, 712, 714 can be denoted as x(n)[i, j].

However, the arrangement of the nodes 720, 722, 724 of one node layer 710, 712, 714 does not have an effect on the calculations executed within the convolutional neural network 700 as such, since these are given solely by the structure and the weights of the edges.

A convolutional layer 711 is a connection layer between an anterior node layer 710 (with node values x(n-1)) and a posterior node layer 712 (with node values x(n)). In particular, a convolutional layer 711 is characterized by the structure and the weights of the incoming edges forming a convolution operation based on a certain number of kernels. In particular, the structure and the weights of the edges of the convolutional layer 711 are chosen such that the values x(n) of the nodes 722 of the posterior node layer 712 are calculated as a convolution x(n) = K * x(n-1) based on the values x(n-1) of the nodes 720 anterior node layer 710, where the convolution * is defined in the two-dimensional case as

$$x_k^{(n)}[i,j] = \left( K * x^{(n-1)} \right)[i,j] = \sum_{i'} \sum_{j'} K[i',j'] \cdot x^{(n-1)}[i-i', j-j'].$$

Here the kernel K is a d-dimensional matrix (in this embodiment, a two-dimensional matrix), which is usually small compared to the number of nodes 720, 722 (e.g., a 3x3 matrix, or a 5x5 matrix). In particular, this implies that the weights of the edges in the convolution layer 711 are not independent, but chosen such that they produce said convolution equation. In particular, for a kernel being a 3x3 matrix, there are only 9 independent weights (each entry of the kernel matrix corresponding to one independent weight), irrespectively of the number of nodes 720, 722 in the anterior node layer 710 and the posterior node layer 712.

In general, convolutional neural networks 700 use node layers 710, 712, 714 with a plurality of channels, in particular, due to the use of a plurality of kernels in convolutional layers 711. In those cases, the node layers can be considered as (d+1)-dimensional matrices (the first dimension indexing the channels). The action of a convolutional layer 711 is then a two-dimensional example defined as

$$x^{(n)_b}[i,j] = \sum_a K_{a,b} * x^{(n-1)_a}[i,j] = \sum_a \sum_{i'} \sum_{j'} K_{a,b}[i',j'] \cdot x^{(n-1)_a}[i-i', j-j']$$

where $x^{(n-1)_a}$ corresponds to the a-th channel of the anterior node layer 710, $x^{(n)_b}$ corresponds to the b-th channel of the posterior node layer 712 and $\kappa_{a,b}$ corresponds to one of the kernels. If a convolutional layer 711 acts on an anterior node layer 710 with A channels and outputs a posterior node layer 712 with B channels, there are A B independent d-dimensional kernels $\kappa_{a,b}$.

In general, in convolutional neural networks 700 activation functions are used. In this embodiment re ReLU (acronym for "Rectified Linear Units") is used, with R(z) = max(0, z), so that the action of the convolutional layer 711 in the two-dimensional example is

$$x^{(n)_b}[i,j] = R\left( \sum_a \left( K_{a,b} * x^{(n-1)_a} \right)[i,j] \right) = R\left( \sum_a \sum_{i'} \sum_{j'} K_{a,b}[i',j'] \cdot x^{(n-1)_a}[i-i', j-j'] \right)$$

It is also possible to use other activation functions, e.g., ELU (acronym for "Exponential Linear Unit"), LeakyReLU, Sigmoid, Tanh or Softmax.

In the displayed embodiment, the input layer 710 comprises 36 nodes 720, arranged as a two-dimensional 6x6 matrix. The first hidden node layer 712 comprises 72 nodes 722, arranged as two two-dimensional 6x6 matrices, each of the two matrices being the result of a convolution of the values of the input layer with a 3x3 kernel within the convolutional layer 711. Equivalently, the nodes 722 of the first hidden node layer 712 can be interpreted as arranged as a three-dimensional 2x6x6 matrix, wherein the first dimension correspond to the channel dimension.

The advantage of using convolutional layers 711 is that spatially local correlation of the input data can exploited by enforcing a local connectivity pattern between nodes of adjacent layers, in particular by each node being connected to only a small region of the nodes of the preceding layer.

A pooling layer 713 is a connection layer between an anterior node layer 712 (with node values x(n-1)) and a posterior node layer 714 (with node values x(n)). In particular, a pooling layer 713 can be characterized by the structure and the weights of the edges and the activation function forming a pooling operation based on a non-linear pooling function f. For example, in the two-dimensional case the values x(n) of the nodes 724 of the posterior node layer 714 can be calculated based on the values x(n-1) of the nodes 722 of the anterior node layer 712 as

$$x^{(n)_b}[i,j] = f(x^{(n-1)_b}[id_1, jd_2], ..., x^{(n-1)_b}[(i+1)d_1-1, (j+1)d_2-1])$$

In other words, by using a pooling layer 713 the number of nodes 722, 724 can be reduced, by re-placing a number d1 d2 of neighboring nodes 722 in the anterior node layer 712 with a single node 722 in the posterior node layer 714 being calculated as a function of the values of said number of neighboring nodes. In particular, the pooling function f can be the max-function, the average or the L2-Norm. In particular, for a pooling layer 713 the weights of the incoming edges are fixed and are not modified by training.

The advantage of using a pooling layer 713 is that the number of nodes 722, 724 and the number of parameters is reduced. This leads to the amount of computation in the network being reduced and to a control of overfitting.

In the displayed embodiment, the pooling layer 713 is a max-pooling layer, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

In general, the last layers of a convolutional neural network 700 are fully connected layers 715. A fully connected layer 715 is a connection layer between an anterior node layer 714 and a posterior node layer 716. A fully connected layer 713 can be characterized by the fact that a majority, in particular, all edges between nodes 714 of the anterior node layer 714 and the nodes 716 of the posterior node layer are present, and wherein the weight of each of these edges can be adjusted individually.

In this embodiment, the nodes 724 of the anterior node layer 714 of the fully connected layer 715 are displayed both as two-dimensional matrices, and additionally as non-related nodes (indicated as a line of nodes, wherein the number of nodes was reduced for a better presentability). This operation is also denoted as "flattening". In this embodiment, the number of nodes 726 in the posterior node layer 716 of the fully connected layer 715 smaller than the number of nodes 724 in the anterior node layer 714. Alternatively, the number of nodes 726 can be equal or larger.

Furthermore, in this embodiment the Softmax activation function is used within the fully connected layer 715. By applying the Softmax function, the sum the values of all nodes 726 of the output layer 716 is 1, and all values of all nodes 726 of the output layer 716 are real numbers between 0 and 1. In particular, if using the convolutional neural network 700 for categorizing input data, the values of the output layer 716 can be interpreted as the probability of the input data falling into one of the different categories.

In particular, convolutional neural networks 700 can be trained based on the backpropagation algorithm. For preventing overfitting, methods of regularization can be used, e.g., dropout of nodes 720, ..., 724, stochastic pooling, use of artificial data, weight decay based on the L1 or the L2 norm, or max norm constraints.

According to an aspect, the machine learning model may comprise one or more residual networks (ResNet). In particular, a ResNet is an artificial neural network comprising at least one jump or skip connection used to jump over at least one layer of the artificial neural network. In particular, a ResNet may be a convolutional neural network comprising one or more skip connections respectively skipping one or more convolutional layers. According to some examples, the ResNets may be represented as m-layer ResNets, where m is the number of layers in the corresponding architecture and, according to some examples, may take values of 34, 50, 101, or 152. According to some examples, such an m-layer ResNet may respectively comprise (m-2)/2 skip connections.

A skip connection may be seen as a bypass which directly feeds the output of one preceding layer over one or more bypassed layers to a layer succeeding the one or more bypassed layers. Instead of having to directly fit a desired mapping, the bypassed layers would then have to fit a residual mapping "balancing" the directly fed output.

Fitting the residual mapping is computationally easier to optimize than the directed mapping. What is more, this alleviates the problem of vanishing/exploding gradients during optimization upon training the machine learning models: if a bypassed layer runs into such problems, its contribution may be skipped by regularization of the directly fed output. Using ResNets thus brings about the advantage that much deeper networks may be trained.

In particular, a recurrent machine learning model is a machine learning model whose output does not only depend on the input value and the parameters of the machine learning model adapted by the training process, but also on a hidden state vector, wherein the hidden state vector is based on previous inputs used on for the recurrent machine learning model. In particular, the recurrent machine learning model can comprise additional storage states or additional structures that incorporate time delays or comprise feedback loops.

In particular, the underlying structure of a recurrent machine learning model can be a neural network, which can be denoted as recurrent neural network. Such a recurrent neural network can be described as an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network).

In particular, training a recurrent neural network can be based on the BPTT algorithm (acronym for "backpropagation through time"), on the RTRL algorithm (acronym for "real-time recurrent learning") and/or on genetic algorithms.

By using a recurrent machine learning model input data comprising sequences of variable length can be used. In particular, this implies that the method cannot be used only for a fixed number of input datasets (and needs to be trained differently for every other number of input datasets used as input), but can be used for an arbitrary number of input datasets. This implies that the whole set of training data, independent of the number of input datasets contained in different sequences, can be used within the training, and that training data is not reduced to training data corresponding to a certain number of successive input datasets.

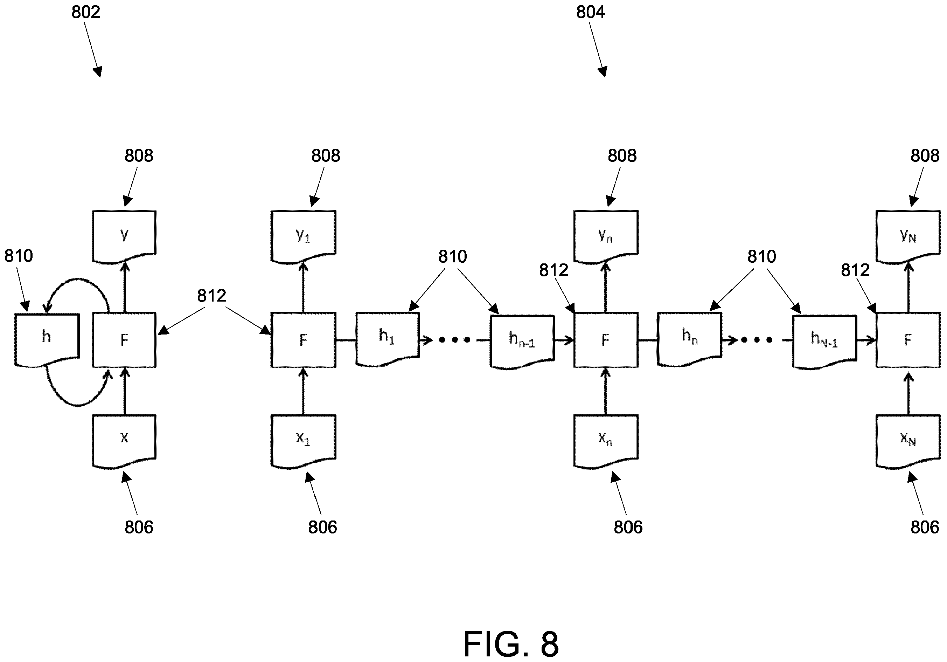

Figure 8 shows the schematic structure of a recurrent machine learning model F, both in a recurrent representation 802 and in an unfolded representation 804, that may be used to implement one or more machine learning models described herein. The recurrent machine learning model takes as input several input datasets $x, x_1, ..., x_N$ 806 and creates a corresponding set of output datasets $y, y_1, ..., y_N$ 808. Furthermore, the output depends on a so-called hidden vector $h, h_1, ..., h_N$ 810, which implicitly comprises information about input datasets previously used as input for the recurrent machine learning model F 812. By using these hidden vectors $h, h_1, ..., h_N$ 810, a sequentiality of the input datasets can be leveraged.

In a single step of the processing, the recurrent machine learning model F 812 takes as input the hidden vector $h_{n-1}$ created within the previous step and an input dataset $x_n$. Within this step, the recurrent machine learning model F generates as output an updated hidden vector $h_n$ and an output dataset $y_n$. In other words, one step of processing calculates $(y_n, h_n) = F(x_n, h_{n-1})$, or by splitting the recurrent machine learning model F 812 into a part F(y) calculating the output data and F(h) calculating the hidden vector, one step of processing calculates $y_n = F^{(y)}(x_n, h_{n-1})$ and $h_n = F^{(h)}(x_n, h_{n-1})$. For the first processing step, $h_0$ can be chosen randomly or filled with all entries being zero. The parameters of the recurrent machine learning model F 812 that were trained based on training datasets before do not change between the different processing steps.

In particular, the output data and the hidden vector of a processing step depend on all the previous input datasets used in the previous steps. $y_n = F^{(y)}(x_n, F^{(h)}(x_{n-1}, h_{n-2}))$ and $h_n = F(h)(x_n, F^{(h)}(X_{n-1}, h_{n-2}))$.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1-5. Certain steps or functions of the methods and workflows described herein, including one or more of the steps or functions of Figures 1-5, may be performed by a server or by another processor in a network-based cloudcomputing system. Certain steps or functions of the methods and workflows described herein, including one or more of the steps of Figures 1-5, may be performed by a client computer in a network-based cloud computing system. The steps or functions of the methods and workflows described herein, including one or more of the steps of Figures 1-5, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1-5, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 902 that may be used to implement systems, apparatuses, and methods described herein is depicted in Figure 9. Computer 902 includes a processor 904 operatively coupled to a data storage device 912 and a memory 910. Processor 904 controls the overall operation of computer 902 by executing computer program instructions that define such operations. The computer program instructions may be stored in data storage device 912, or other computer readable medium, and loaded into memory 910 when execution of the computer program instructions is desired. Thus, the method and workflow steps or functions of Figures 1-5 can be defined by the computer program instructions stored in memory 910 and/or data storage device 912 and controlled by processor 904 executing the computer program instructions. For example, the computer program instructions can be implemented as computer executable code programmed by one skilled in the art to perform the method and workflow steps or functions of Figures 1-5. Accordingly, by executing the computer program instructions, the processor 904 executes the method and workflow steps or functions of Figures 1-5. Computer 902 may also include one or more network interfaces 906 for communicating with other devices via a network. Computer 902 may also include one or more input/output devices 908 that enable user interaction with computer 902 (e.g., display, keyboard, mouse, speakers, buttons, etc.).

Processor 904 may include both general and special purpose microprocessors, and may be the sole processor or one of multiple processors of computer 902. Processor 904 may include one or more central processing units (CPUs), for example. Processor 904, data storage device 912, and/or memory 910 may include, be supplemented by, or incorporated in, one or more application-specific integrated circuits (ASICs) and/or one or more field programmable gate arrays (FPGAs).

Data storage device 912 and memory 910 each include a tangible non-transitory computer readable storage medium. Data storage device 912, and memory 910, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 908 may include peripherals, such as a printer, scanner, display screen, etc. For example, input/output devices 908 may include a display device such as a cathode ray tube (CRT) or liquid crystal display (LCD) monitor for displaying information to the user, a keyboard, and a pointing device such as a mouse or a trackball by which the user can provide input to computer 902.

An image acquisition device 914 can be connected to the computer 902 to input image data (e.g., medical images) to the computer 902. It is possible to implement the image acquisition device 914 and the computer 902 as one device. It is also possible that the image acquisition device 914 and the computer 902 communicate wirelessly through a network. In a possible embodiment, the computer 902 can be located remotely with respect to the image acquisition device 914.

Any or all of the systems, apparatuses, and methods discussed herein may be implemented using one or more computers such as computer 902.

One skilled in the art will recognize that an implementation of an actual computer or computer system may have other structures and may contain other components as well, and that Figure 9 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope and spirit of the invention. Those skilled in the art could implement various other feature combinations without departing from the scope and spirit of the invention.

The following is a list of non-limiting illustrative embodiments disclosed herein:

Illustrative embodiment 1. A computer-implemented method comprising: receiving text-based instructions; encoding the text-based instructions into text features using a machine learning based text encoder network; determining one or more actions for performing on one or more medical applications using a policy module based on the text features; performing the one or more actions on the one or more medical applications to generate a response to the text-based instructions; and outputting the response to the text-based instructions.

Illustrative embodiment 2. The computer-implemented method of illustrative embodiment 1, further comprising: receiving one or more medical images; and encoding the one or more medical images into image features using a machine learning based image encoder network, wherein determining one or more actions for performing on one or more medical applications using a policy module based on the text features comprises determining the one or more actions further based on the image features.

Illustrative embodiment 3. The computer-implemented method of illustrative embodiment 2, wherein: performing the one or more actions on the one or more medical applications to generate a response to the text-based instructions comprises performing the one or more actions on the one or more medical applications to modify the one or more medical images, and outputting the response to the text-based instructions comprises outputting the one or more modified medical images.

Illustrative embodiment 4. The computer-implemented method of any one of illustrative embodiments 2-3, wherein the machine learning based text encoder network and the machine learning based image encoder network are trained to generate similar features for associated text-based instructions and medical images.

Illustrative embodiment 5. The computer-implemented method of any one of illustrative embodiments 1-4, further comprising: adapting the policy module based on user feedback to the response to the text-based instructions.

Illustrative embodiment 6. The computer-implemented method of any one of illustrative embodiments 1-5, wherein the one or more actions comprise at least one of: one or more medical image analysis tasks performed on one or more medical images, functions to derive findings from the one or more medical images, functions to apply transformations on the one or more medical images, functions to derive information from the one or more medical images, or outputting text to a machine learning based model.

Illustrative embodiment 7. The computer-implemented method of any one of illustrative embodiments 1-6, wherein the one or more actions comprise one or more APIs (application programming interfaces).

Illustrative embodiment 8. The computer-implemented method of any one of illustrative embodiments 1-7, wherein receiving text-based instructions comprises: receiving spoken instructions from a user; and converting the spoken instructions to the text-based instructions.

Illustrative embodiment 9. The computer-implemented method of any one of illustrative embodiments 1-8, wherein the machine learning based text encoder network comprises a language model.

Illustrative embodiment 10. An apparatus comprising: means for receiving text-based instructions; means for encoding the text-based instructions into text features using a machine learning based text encoder network; means for determining one or more actions for performing on one or more medical applications using a policy module based on the text features; means for performing the one or more actions on the one or more medical applications to generate a response to the text-based instructions; and means for outputting the response to the text-based instructions.

Illustrative embodiment 11. The apparatus of illustrative embodiment 10, further comprising: means for receiving one or more medical images; and means for encoding the one or more medical images into image features using a machine learning based image encoder network, wherein the means for determining one or more actions for performing on one or more medical applications using a policy module based on the text features comprises means for determining the one or more actions further based on the image features.

Illustrative embodiment 12. The apparatus of illustrative embodiment 11, wherein: the means for performing the one or more actions on the one or more medical applications to generate a response to the text-based instructions comprises means for performing the one or more actions on the one or more medical applications to modify the one or more medical images, and the means for outputting the response to the text-based instructions comprises means for outputting the one or more modified medical images.

Illustrative embodiment 13. The apparatus of claim any one of illustrative embodiments 11-12, wherein the machine learning based text encoder network and the machine learning based image encoder network are trained to generate similar features for associated text-based instructions and medical images.

Illustrative embodiment 14. The apparatus of any one of illustrative embodiments 10-13, further comprising: means for adapting the policy module based on user feedback to the response to the text-based instructions.

Illustrative embodiment 15. A non-transitory computer-readable storage medium comprising instructions which, when executed by a computer, cause the computer to carry out operations comprising: receiving text-based instructions; encoding the text-based instructions into text features using a machine learning based text encoder network; determining one or more actions for performing on one or more medical applications using a policy module based on the text features; performing the one or more actions on the one or more medical applications to generate a response to the text-based instructions; and outputting the response to the text-based instructions.

Illustrative embodiment 16. The non-transitory computer-readable storage medium of illustrative embodiment 15, the operations further comprising: receiving one or more medical images; and encoding the one or more medical images into image features using a machine learning based image encoder network, wherein determining one or more actions for performing on one or more medical applications using a policy module based on the text features comprises determining the one or more actions further based on the image features.

Illustrative embodiment 17. The non-transitory computer-readable storage medium of any one of illustrative embodiments 15-16, wherein the one or more actions comprise at least one of: one or more medical image analysis tasks performed on one or more medical images, functions to derive findings from the one or more medical images, functions to apply transformations on the one or more medical images, functions to derive information from the one or more medical images, or outputting text to a machine learning based model.

Illustrative embodiment 18. The non-transitory computer-readable storage medium of any one of illustrative embodiments 15-17, wherein the one or more actions comprise one or more APIs (application programming interfaces).

Illustrative embodiment 19. The non-transitory computer-readable storage medium of any one of illustrative embodiments 15-18, wherein receiving text-based instructions comprises: receiving spoken instructions from a user; and converting the spoken instructions to the text-based instructions.

Illustrative embodiment 20. The non-transitory computer-readable storage medium of any one of illustrative embodiments 15-19, wherein the machine learning based text encoder network comprises a language model.

**Claims**

1. A computer-implemented method comprising:

   receiving (102) text-based instructions (204);
   encoding (104) the text-based instructions into text features using a machine learning based text encoder network (210, 304, 404, 504);
   determining (108) one or more instructions for performing by one or more medical applications using a policy module (212, 406, 506) based on the text features;
   performing (110) the one or more instructions by the one or more medical applications to generate a response (214, 216) to the text-based instructions; and
   outputting (112) the response to the text-based instructions.

2. The computer-implemented method of claim 1, further comprising:

   receiving (102) one or more medical images (202); and
   encoding (106) the one or more medical images into image features using a machine learning based image encoder network (208, 302, 402, 502),
   wherein determining one or more instructions for performing by one or more medical applications using a policy module based on the text features comprises determining the one or more actions further based on the image features.

3. The computer-implemented method of claim 2, wherein:

   performing the one or more instructions by the one or more medical applications to generate a response to the text-based instructions comprises performing the one or more instructions the one or more medical applications to modify the one or more medical images, and
   outputting the response to the text-based instructions comprises outputting the one or more modified medical images.

4. The computer-implemented method of claim 2, wherein the machine learning based text encoder network and the machine learning based image encoder network are trained to generate similar features (306) for associated text-based instructions and medical images.

5. The computer-implemented method of claim 1, further comprising:
   adapting (510) the policy module based on user feedback (508) to the response to the text-based instructions.

6. The computer-implemented method of claim 1, wherein the one or more instructions comprise at least one of: one or more medical image analysis tasks performed on one or more medical images, functions to derive findings from the one or more medical images, functions to apply transformations on the one or more medical images, functions to derive information from the one or more medical images, or outputting text to a machine learning based model.

7. The computer-implemented method of claim 1, wherein the one or more instructions comprise one or more APIs (application programming interfaces).

8. The computer-implemented method of claim 1, wherein receiving text-based instructions comprises:

   receiving spoken instructions from a user; and

converting the spoken instructions to the text-based instructions.

9. The computer-implemented method of claim 1, wherein the machine learning based text encoder network comprises a language model.

10. An apparatus comprising:

   means for receiving (102) text-based instructions (204);
   means for encoding (104) the text-based instructions into text features using a machine learning based text encoder network (210, 304, 404, 504);
   means for determining (108) one or more instructions for performing by one or more medical applications using a policy module (212, 406, 506) based on the text features;
   means for performing (110) the one or more instructions by the one or more medical applications to generate a response (214, 216) to the text-based instructions; and
   means for outputting (112) the response to the text-based instructions.

11. The apparatus of claim 10, further comprising:

   means for receiving (102) one or more medical images (202); and
   means for encoding (106) the one or more medical images into image features using a machine learning based image encoder network (208, 302, 402, 502),
   wherein the means for determining one or more instructions for performing by one or more medical applications using a policy module based on the text features comprises means for determining the one or more actions further based on the image features.

12. The apparatus of claim 11, wherein:

   the means for performing the one or more instructions by the one or more medical applications to generate a response to the text-based instructions comprises means for performing the one or more instructions by the one or more medical applications to modify the one or more medical images, and
   the means for outputting the response to the text-based instructions comprises means for outputting the one or more modified medical images.

13. The apparatus of claim 11, wherein the machine learning based text encoder network and the machine learning based image encoder network are trained to generate similar features (306) for associated text-based instructions and medical images.

14. The apparatus of claim 10, further comprising:
   means for adapting (510) the policy module based on user feedback (508) to the response to the text-based instructions.

15. A non-transitory computer-readable storage medium comprising instructions which, when executed by a computer, cause the computer to carry out operations comprising:

   receiving (102) text-based instructions (204);
   encoding (104) the text-based instructions into text features using a machine learning based text encoder network (210, 304, 404, 504);
   determining (108) one or more instructions for performing by one or more medical applications using a policy module (212, 406, 506) based on the text features;
   performing (110) the one or more instructions by the one or more medical applications to generate a response (214, 216) to the text-based instructions; and
   outputting (112) the response to the text-based instructions.

16. The non-transitory computer-readable storage medium of claim 15, the operations further comprising:

   receiving (102) one or more medical images (202); and
   encoding (106) the one or more medical images into image features using a machine learning based image encoder network (208, 302, 402, 502),

wherein determining one or more instructions for performing by one or more medical applications using a policy module based on the text features comprises determining the one or more actions further based on the image features.

17. The non-transitory computer-readable storage medium of claim 15, wherein the one or more instructions comprise at least one of: one or more medical image analysis tasks performed on one or more medical images, functions to derive findings from the one or more medical images, functions to apply transformations on the one or more medical images, functions to derive information from the one or more medical images, or outputting text to a machine learning based model.

18. The non-transitory computer-readable storage medium of claim 15, wherein the one or more instructions comprise one or more APIs (application programming interfaces).

19. The non-transitory computer-readable storage medium of claim 15, wherein receiving text-based instructions comprises:

receiving spoken instructions from a user; and
converting the spoken instructions to the text-based instructions.

20. The non-transitory computer-readable storage medium of claim 15, wherein the machine learning based text encoder network comprises a language model.

# FIG. 1

<u>100</u>

Receive text-based instructions and one or more medical images
<u>102</u>

Encode the text-based instructions into text features using a machine learning based text encoder network
<u>104</u>

Encode the one or more medical images into image features using a machine learning based image encoder network
<u>106</u>

Determine one or more instructions for performing by one or more medical applications using a policy module based on the text features and the image features
<u>108</u>

Perform the one or more instructions by the one or more medical applications to generate a response to the text-based instructions
<u>110</u>

Output the response to the text-based instructions
<u>112</u>

## 200

FIG. 2

# FIG. 3

<u>300</u>

306

302 → Image Encoder

304 → Large Language Model

Similar representations

# FIG. 4

<u>400</u>

# FIG. 5

500

510

Adaptation

502

Image Encoding
(Optional)

506

User feedback

Text Encoding

Fine-tuning
policy

508

504

Output

512

FIG. 6

EP 4 718 468 A1

FIG. 7

FIG. 8

# FIG. 9

# EUROPEAN SEARCH REPORT

**Application Number**

EP 24 46 5579

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/156921 A1 (KOHLI NAMITA [US] ET AL) 23 May 2019 (2019-05-23)<br>* figures 4,10 *<br>* paragraph [0067] *<br>* paragraph [0100] - paragraph [0117] *<br>* paragraph [0140] - paragraph [0143] *<br>* paragraph [0159] *<br>* paragraph [0167] *<br>----- | 1-20 | INV.<br>G16H50/20<br>G16H30/20 |
| X | US 2021/240931 A1 (FARRI OLADIMEJI FEYISETAN [US] ET AL) 5 August 2021 (2021-08-05)<br>* figures 1-4 *<br>* paragraph [0004] - paragraph [0017] *<br>----- | 1-20 | |
| A | WO 2024/077082 A1 (HOME DEPOT INT INC [US]) 11 April 2024 (2024-04-11)<br>* paragraph [0017] - paragraph [0019] *<br>* paragraph [0038] - paragraph [0051] *<br>* paragraph [0063] *<br>----- | 1-20 | |
| A | US 11 875 128 B2 (ADA SUPPORT INC [CA]) 16 January 2024 (2024-01-16)<br>* the whole document *<br>----- | 1-20 | **TECHNICAL FIELDS SEARCHED (IPC)**<br><br>G16H |

The present search report has been drawn up for all claims

| Place of search | Date of completion of the search | Examiner |
|---|---|---|
| Munich | 11 March 2025 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 24 46 5579

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2025

| Patent document cited in search report | | Publication date | Patent family member(s) | | Publication date |
|---|---|---|---|---|---|
| US 2019156921 | A1 | 23-05-2019 | US | 2019156921 A1 | 23-05-2019 |
| | | | WO | 2019104093 A1 | 31-05-2019 |
| US 2021240931 | A1 | 05-08-2021 | EP | 3788632 A1 | 10-03-2021 |
| | | | US | 2021240931 A1 | 05-08-2021 |
| | | | WO | 2019211250 A1 | 07-11-2019 |
| WO 2024077082 | A1 | 11-04-2024 | US | 2025037010 A1 | 30-01-2025 |
| | | | WO | 2024077082 A1 | 11-04-2024 |
| US 11875128 | B2 | 16-01-2024 | NONE | | |

EPO FORM P0459

For more details about this annex : see Official Journal of the European Patent Office, No. 12/82